# EUROPÄISCHE PATENTANMELDUNG

(11) **EP 1 428 490 A1**
(43) Veröffentlichungstag der Anmeldung: **16.06.2004**
(21) Anmeldenummer: 02102725.5
(22) Anmeldetag: 11.12.2002
(51) Int. Cl.: A61H 9/00, A61M 1/08

(54) **Körperpflegegerät mit einer Saugpipette**

(71) Anmelder: Koninklijke Philips Electronics N.V., 5621 BA Eindhoven (NL)
(72) Erfinder: Die Erfindernennung liegt noch nicht vor

(57) **Zusammenfassung**

Bei einem Körperpflegegerät (1) mit einer Luftpumpe (3) und mit einem Saugstück (8) ist das Saugstück (8) rohrförmig ausgebildet und mit nur einer einzigen umfangseitig begrenzten Saugöffnung (13) ausgerüstet, wobei das Saugstück (8) mindestens zwei bis zu der Saugöffnung (13) sich erstreckende Saugstückabschnitte (14, 15) aufweist, die in radialen Richtungen verstellbar und zum Ausüben einer Kraftwirkung auf einen beim Saugen gebildeten Hauthöcker ausgebildet sind, und wobei das Saugstück (8) mindestens zwei ebenso bis zu der Saugöffnung (13) sich erstreckende und die Saugöffnung (13) begrenzende und mit den zwei Saugstückabschnitten (14, 15) luftdicht verbundene und elastisch nachgiebig ausgebildete Dichtungsteile (19, 20) aufweist.

## Beschreibung

Die Erfindung bezieht sich auf ein Körperpflegegerät mit einer Luftpumpe und mit einem Saugstück zum Ausüben einer Saugwirkung auf die Haut eines Menschen, wobei das Saugstück mit der Luftpumpe luftleitend verbunden ist und wobei das Saugstück mindestens eine umfangsseitig begrenzte Saugöffnung zum saugenden Zusammenwirken mit der Haut eines Menschen aufweist.

Die Erfindung bezieht sich weiters auf ein Saugstück für ein Körperpflegegerät, wobei das Saugstück mindestens eine umfangsseitig begrenzte Saugöffnung zum saugenden Zusammenwirken mit der Haut eines Menschen aufweist.

Ein solches Körperpflegegerät entsprechend der eingangs in dem ersten Absatz angeführten Gattung und ein solches Saugstück entsprechend der eingangs in dem zweiten Absatz angeführten Gattung sind aus dem Patentdokument EP 0 997 156 A2 bekannt. Bei dem bekannten Körperpflegegerät und bei dem bekannten Saugstück ist die Ausbildung des Saugstücks so getroffen, dass das gesamte Saugstück aus einem einzigen relativ harten Kunststoffmaterial besteht und im Bereich der Saugöffnung einen umfangsseitig in sich geschlossenen hohlzylindrischen Rohrteil aufweist, der in radialen Richtungen praktisch undeformierbar ist und auf diese Weise eine in radialen Richtungen starre Saugkappe bildet. Hierdurch ist der Sachverhalt gegeben, dass das Reinigen der zu behandelnden Haut nur durch die mit Hilfe der Luftpumpe erzeugte Saugwirkung erfolgt. Hierdurch können zwar an der Hautoberfläche befindliche Hautunreinheiten und in der Nähe der offenen Enden von Poren der Haut befindliche Hautunreinheiten relativ gut beseitigt werden, jedoch können in Poren der Haut tieferliegende Verunreinigungen nur unzufriedenstellend bzw. überhaupt nicht entfernt werden, dies deshalb, weil für ein solches Entfernen von in Poren der Haut tieferliegenden Verunreinigungen bei dem bekannten Körperpflegegerät ein verhältnismäßig hoher Unterdruck erforderlich wäre, der aber zu einem unerwünscht hohen Schmerzempfinden und sogar zu Hautverletzungen führen würde.

Die Erfindung hat sich zur Aufgabe gestellt, die vorstehend angeführten Probleme zu beseitigen und ein verbessertes Körperpflegegerät und ein verbessertes Saugstück für ein Körperpflegegerät zu realisieren.

Zur Lösung der vorstehend angeführten Aufgabe sind bei einem Körperpflegegerät gemäß der Erfindung erfindungsgemäße Merkmale vorgesehen, so dass ein Körperpflegegerät gemäß der Erfindung auf die nachfolgend angegebene Weise charakterisierbar ist, nämlich:

Körperpflegegerät mit einer Luftpumpe und mit einem Saugstück zum Ausüben einer Saugwirkung auf die Haut eines Menschen, wobei das Saugstück mit der Luftpumpe luftleitend verbunden ist und wobei das Saugstück mindestens eine umfangsseitig begrenzte Saugöffnung zum saugenden Zusammenwirken mit der Haut eines Menschen aufweist und wobei das Saugstück im Bereich der Saugöffnung zum Bilden eines Hauthöckers bei einem saugenden Zusammenwirken mit der Haut ausgebildet ist und wobei das Saugstück mindestens zwei bis zu der Saugöffnung sich erstreckende und die Saugöffnung begrenzende Saugstückabschnitte aufweist, welche zwei Saugstückabschnitte in radialen Richtungen verstellbar und zum Ausüben einer Kraftwirkung in radialen Richtungen auf einen bei einem saugenden Zusammenwirken mit der Haut gebildeten Hauthöcker ausgebildet sind, und wobei das Saugstück mindestens zwei bis zu der Saugöffnung sich erstreckende und die Saugöffnung begrenzende und elastisch nachgiebig ausgebildete Dichtungsteile aufweist, wobei jeder Dichtungsteil zwischen zwei zueinander benachbarten Saugstückabschnitten liegt und mit den zwei zueinander benachbarten Saugstückabschnitten luftdicht verbunden ist.

Zur Lösung der vorstehend angeführten Aufgabe sind bei einem Saugstück gemäß der Erfindung erfindungsgemäße Merkmale vorgesehen, so dass ein Saugstück gemäß der Erfindung auf die nachfolgend angegebene Weise charakterisierbar ist, nämlich:

Saugstück für ein Körperpflegegerät, wobei das Saugstück mindestens eine umfangsseitig begrenzte Saugöffnung zum saugenden Zusammenwirken mit der Haut eines Menschen aufweist und wobei das Saugstück im Bereich der Saugöffnung zum Bilden eines Hauthöckers bei einem saugenden Zusammenwirken mit der Haut ausgebildet ist und wobei das Saugstück mindestens zwei bis zu der Saugöffnung sich erstreckende und die Saugöffnung begrenzende Saugstückabschnitte aufweist, welche zwei Saugstückabschnitte in radialen Richtungen verstellbar und zum Ausüben einer Kraftwirkung in radialen Richtungen auf einen bei einem saugenden Zusammenwirken mit der Haut gebildeten Hauthöcker ausgebildet sind, und wobei das Saugstück mindestens zwei bis zu der Saugöffnung sich erstreckende und die Saugöffnung begrenzende und elastisch nachgiebig ausgebildete Dichtungsteile aufweist, wobei jeder Dichtungsteil zwischen zwei zueinander benachbarten Saugstückabschnitten liegt und mit den zwei zueinander benachbarten Saugstückabschnitten luftdicht verbunden ist.

Durch das Vorsehen der Merkmale gemäß der Erfindung ist auf baulich einfache Weise und mit einem nur geringen Zusatzaufwand erreicht, dass mit einem Saugstück gemäß der Erfindung nicht nur eine Saugwirkung auf die Haut eines Menschen ausgeübt werden kann, sondern dass zusätzlich auch noch bei einem saugenden Zusammenwirken des Saugstücks mit der Haut auf den hierbei durch die Saugwirkung gebildeten Hauthöcker eine Kraftwirkung von der Seite her ausgeübt werden kann. Durch das Ausüben einer solchen Kraftwirkung wird ein Herausdrücken von in Poren der Haut tieferliegenden Verunreinigungen unterstützt bzw. bewerkstelligt, so dass solche ursprünglich tieferliegenden Verunreinigungen nach ihrem Herausdrücken in Richtung zu den Enden der Poren auf Grund der ausgeübten Saugwirkung leichter entfernt werden können. Hierbei ist bei dem Saugstück gemäß der Erfindung der große Vorteil sichergestellt, dass mit Hilfe der luftdicht mit den mindestens zwei verstellbaren Saugstückabschnitten verbundenen Dichtungsteile ein vollkommen dichtes Verhalten des Saugstücks erreicht ist, so dass keinerlei Verlust an Saugwirkung im Bereich der Saugöffnung auftreten kann.

Es sei erwähnt, dass aus dem Patentdokument US 5 624 416 A ein Körperpflegegerät mit einer Luftpumpe und mit einer Saugstückkonfiguration bekannt ist. Bei diesem Körperpflegegerät weist die Saugstückkonfiguration eine Mehrzahl von Saugöffnungen auf. Bei dem bekannten Körperpflegegerät sind im Bereich jeder Saugöffnung zwei einander gegenüberliegende und zueinander verstellbare Saugstückteile zum Ausüben einer Kraftwirkung von der Seite her auf einen Hauthöcker vorgesehen, jedoch sind die zwei Saugstückteile im Bereich jeder der mehreren Saugöffnungen nicht mit Hilfe von luftdicht mit den Saugstückteilen verbundenen Dichtungsteilen miteinander verbunden, was leider zur Folge hat, dass die bei dem bekannten Gerät mit Hilfe der Luftpumpe erzeugte Saugwirkung, also Unterdruckwirkung, nicht zur Gänze auf die zu behandelnde Haut aufgebracht werden kann, weil auf Grund der undichten Ausbildung der Saugstückkonfiguration im Bereich der Saugöffnungen es zu einem erheblichen Verlust an Unterdruckwirkung kommt, was wiederum nachteilig für ein möglichst gutes Reinigen der zu behandelnden Haut ist.

Bei einem Körperpflegegerät gemäß der Erfindung und bei einem Saugstück gemäß der Erfindung kann vorgesehen sein, dass das Saugstück zumindest in dem die Saugöffnung aufweisenden Bereich aus einem Rohrteil aus einem elastisch deformierbaren Kunststoff oder einem elastisch deformierbaren Gummi besteht, wobei der Rohrteil mit Hilfe von zwei Fingern einer Hand, beispielsweise mit Hilfe von einem Daumen und dem benachbarten Zeigefinger, zusammengedrückt werden kann, wobei dann die mit Hilfe der beiden Finger zusammendrückbaren Abschnitte des Rohrteils die in radialen Richtungen verstellbaren Saugstückabschnitte bilden und wobei die gegenüber den beiden Fingern frei liegenden Bereiche des Rohrteils die elastisch nachgiebig ausgebildeten Dichtungsteile des Saugstücks bilden. Als besonders vorteilhaft hat es sich aber erwiesen, wenn das Saugstück zwei einander diametral gegenüberliegende Saugstückabschnitte aufweist, die aus einem in Relation zu den elastisch nachgiebig ausgebildeten Dichtungsteilen harten Material bestehen. Hierdurch ist der Vorteil gegeben, dass die zum Ausüben einer Kraftwirkung auf einen Hauthöcker vorgesehenen Saugstückabschnitte auf Grund ihrer relativ hohen Härte zum Aufbringen von relativ großen Kräften geeignet sind, was im Hinblick auf das Erzeugen einer möglichst hohen Kraftwirkung auf einen Hauthöcker vorteilhaft ist.

Es sei aber erwähnt, dass bei einem Saugstück gemäß der Erfindung auch drei oder vier Saugstückabschnitte aus einem relativ harten Material vorgesehen sein können, welche Saugstückabschnitte mit Hilfe von beispielsweise einem über die Saugstückabschnitte gestülpten Verstellring zueinander verstellbar sind, welche Lösung vom Prinzip her ähnlich ist, wie sie bei einem sogenannten Bohrfutter zum Einspannen eines Bohrers bei einer Bohrmaschine bekannt ist.

Bei einem Körperpflegegerät gemäß der Erfindung und bei einem Saugstück gemäß der Erfindung können die mindestens zwei elastisch nachgiebig ausgebildeten Dichtungsteile je durch einen separaten Gummiteil gebildet sein, der mit Hilfe von Klebeverbindungen mit den zueinander benachbarten Saugstückteilen luftdicht verbunden sind. Als besonders vorteilhaft hat es sich aber erwiesen, wenn das Saugstück mit seinen zwei Saugstückteilen und seinen zwei Dichtungsteilen mit Hilfe von einem Zwei-Komponenten-Spritzgießverfahren hergestellt worden ist. Eine solche Ausbildung ist im Hinblick auf eine möglichst einfache bauliche Ausbildung und eine möglichst einfache Herstellbarkeit und im Hinblick auf ein einfaches luftdichtes Verbinden der Dichtungsteile mit den Saugstückteilen vorteilhaft. Anstelle eines Zwei-Komponenten-Spritzgießverfahren kann aber auch ein Mehr-Komponenten-Spritzgießverfahren angewendet werden. Auch kann ein auf diese Weise hergestelltes Saugstück auch mehr als zwei verstellbare Saugstückabschnitte und dementsprechend mehr als zwei Dichtungsteile aufweisen.

Bei einem Körperpflegegerät gemäß der Erfindung und bei einem Saugstück gemäß der Erfindung hat es sich weiters als sehr vorteilhaft erwiesen, wenn die mindestens zwei Saugstückteile des Saugstücks je eine scharfe Begrenzungskante zum Begrenzen der Saugöffnung aufweisen. Durch das Vorsehen solcher scharfen Begrenzungskanten wird eine gute Schabwirkung erreicht, wodurch eine gute Reinigungswirkung erzielt wird. In diesem Zusammenhang hat es sich weiters als sehr vorteilhaft erwiesen, wenn auch die zwei mindestens Dichtungsteile des Saugstücks je eine scharfe Begrenzungskante zum Begrenzen der Saugöffnung aufweisen. Es sei erwähnt, dass die mindestens zwei Saugstückteile des Saugstücks und die mindestens zwei Dichtungsteile des Saugstücks aber auch je eine leicht abgerundete Begrenzungskante zum Begrenzen der Saugöffnung aufweisen können, wodurch dann ein guter Kompromiss zwischen einer möglichst guten Schabwirkung einerseits und einem möglichst schonenden Behandeln der Haut andererseits erreicht wird.

Bei einem wie in dem vorstehenden Absatz angeführten Körperpflegegerät und bei einem wie in dem vorstehenden Absatz angeführten Saugstück hat es sich als besonders vorteilhaft erwiesen, wenn die mindestens zwei Begrenzungskanten der Saugstückteile einen kreisbogenförmigen Verlauf aufweisen. Hierbei hat es sich weiters als sehr vorteilhaft erwiesen, wenn auch die mindestens zwei Begrenzungskanten der Dichtungsteile einen kreisbogenförmigen Verlauf aufweisen. Hierdurch ist eine Saugöffnung mit einem kreisförmigen Querschnitt erreicht, was ist im Hinblick auf ein möglichst geringes Verschmutzen des Saugstücks im Bereich der Saugöffnung vorteilhaft ist. Es sei erwähnt, dass aber auch andere Saugöffnungen mit anderen Querschnittsformen möglich sind, beispielsweise mit einer ovalen oder einer quadratischen oder einer sechseckigen Querschnittsform.

Bei den in dem vorstehenden Absatz angeführten Lösungen hat es sich weiters als sehr vorteilhaft erwiesen, wenn die mindestens zwei einen kreisbogenförmigen Verlauf aufweisenden Begrenzungskanten einen diametrischen Abstand in einem Bereich zwischen 3,0 mm und 4,0 mm aufweisen, wobei es sich als besonders vorteilhaft erwiesen hat, wenn die mindestens zwei Begrenzungskanten einen diametrischen Abstand von 3,4 mm aufweisen. Eine solche Ausbildung hat sich in der Praxis als besonders vorteilhaft im Hinblick auf gute Hautreinigungsergebnisse erwiesen, insbesondere dann, wenn in dem Bereich der Saugöffnung des Saugstücks ein Unterdruck von etwa 500 mbar erzeugt wird.

Die vorstehend angeführten Aspekte und weitere Aspekte der Erfindung gehen aus dem nachfolgend beschriebenen Ausführungsbeispiel hervor und sind anhand dieses Ausführungsbeispiels erläutert.

Die Erfindung wird im Folgenden anhand von einem in der Zeichnung dargestellten Ausführungsbeispiel weiter beschrieben, auf das die Erfindung aber nicht beschränkt ist.

Die Figur 1 zeigt in einer Schrägansicht ein Körperpflegegerät gemäß einem Ausführungsbeispiel der Erfindung in einem noch nicht zusammengebauten Zustand.

Die Figur 2 zeigt in einem Längsschnitt ein Saugstück des Körperpflegegeräts gemäß der Figur 1.

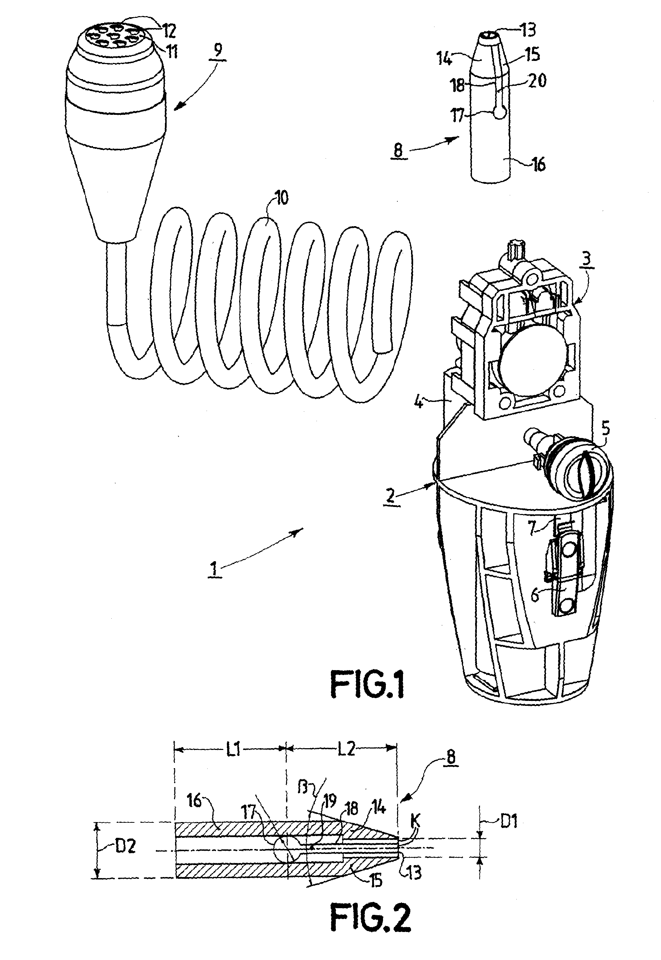

Die Figur 1 zeigt ein Körperpflegegerät 1 in einem noch nicht zusammengebauten Zustand. Hierbei ist in der Figur 1 das Gehäuse des Körperpflegegeräts 1, das aus zwei halbschalenförmigen Gehäuseteilen besteht, nicht dargestellt.

Das Körperpflegegerät 1 weist innerhalb des nicht dargestellten Gehäuses ein Gerätechassis 2 auf, an dem eine Mehrzahl von Geräteteilen und Gerätebaueinheiten befestigt sind. An dem Gerätechassis 2 ist eine Luftpumpe 3 befestigt, die durch eine sogenannte Membranpumpe gebildet ist. Solche Membranpumpen sind seit langem bekannt, weshalb hier nicht näher auf die Ausbildung der Membranpumpe, also der Luftpumpe 3, eingegangen ist. Zum Antreiben der Luftpumpe 3 ist eine Motor 4 vorgesehen, der ebenfalls mit dem Gerätechassis 2 verbunden ist. Mit dem Gerätechassis 2 ist weiters ein Drehschalter 5 verbunden. Der Drehschalter 5 dient zum Einschalten des Motors 4 und weiters zum regelbaren Einstellen des mit Hilfe der Luftpumpe 3 erzeugbaren Unterdrucks. Aus der Figur 1 ist weiters ein elektrischer Schalter 6 ersichtlich, der zum elektrischen Ein- und Ausschalten des Motors 4 vorgesehen ist und der mit Hilfe eines mit dem Drehschalter 5 verstellbaren Betätigungsarms 7 betätigbar ist.

Das Körperpflegegerät 1 ist mit einem ersten Saugstück 8 und mit einem zweiten Saugstück 9 ausgerüstet. Die zwei Saugstücke 8 und 9 sind gegeneinander auswechselbar. Jedes der zwei Saugstücke 8 und 9 kann mit der Luftpumpe 3 luftleitend verbunden werden, und zwar über einen Schlauch 10, der einerseits wahlweise mit dem jeweils zur Verwendung vorgesehenen Saugstück 8 bzw. 9 luftdicht und wiederum lösbar verbunden werden kann und der andererseits mit einem aus der Figur 1 nicht ersichtlichen Anschlussstutzen der Luftpumpe 3 luftdicht und fix verbunden ist. Jedes der zwei Saugstücke 8 und 9 ist zum Ausüben einer Saugwirkung auf die Haut eines Menschen vorgesehen und ausgebildet.

Das zweite Saugstück 9 ist zum Ausüben einer Saugwirkung auf Hautbereiche eines Menschen vorgesehen, die in einem relativ großen Flächenbereich eine relativ geringe Krümmung aufweisen, beispielsweise im Wangenbereich und im Backenbereich und eventuell auch im Kinnbereich. Aus diesem Grund weist das zweite Saugstück 9 eine relativ große Abschlussfläche 11 auf, in der eine Mehrzahl von Saugöffnungen 12 vorgesehen sind.

Das erste Saugstück 8 ist zum Ausüben einer Saugwirkung auf Hautbereiche eines Menschen vorgesehen, welche Hautbereiche in einem relativ kleinen Flächenbereich eine relativ starke Krümmung aufweisen, beispielsweise im Nasenbereich und im Nasenwurzelbereich und eventuell auch im Kinnbereich. Das erste Saugstück 8, das in der Figur 2 in einem Längsschnitt dargestellt ist, ist in diesem Fall rohrförmig ausgebildet und weist nur eine einzige umfangsseitig begrenzte Saugöffnung 13 zum saugenden Zusammenwirken mit der Haut eines Menschen auf. Das erste Saugstück muss aber nicht unbedingt rohrförmig ausgebildet sein, sondern kann auch eine Kugelform oder eine Eiform oder eine Kegelform aufweisen. Auch kann ein solches Saugstück zwei nebeneinanderliegende Saugöffnungen aufweisen. Das rohrförmig ausgebildete erste Saugstück 8 ist im Bereich der Saugöffnung 13 zum Bilden eines in den Figuren 1 und 2 nicht dargestellten Hauthöckers bei einem saugenden Zusammenwirken des ersten Saugstücks 8 mit der Haut eines Menschen ausgebildet. Ein solcher Hauthöcker ragt auf Grund der Saugwirkung von der Saugöffnung 13 her in den an die Saugöffnung 13 anschließenden Innenraum des ersten Saugstücks 8 hinein.

Das erste Saugstück 8 weist zwei bis zu der Saugöffnung 13 sich erstreckende und die Saugöffnung 13 begrenzende und aneinander gegenüberliegende Saugstückabschnitte 14 und 15 auf. Die zwei Saugstückabschnitte 14 und 15 stehen hierbei von einem eine erste Länge L1 aufweisenden Basisteil 16 des ersten Saugstücks 8 ab, wobei sich die zwei Saugstückabschnitte 14 und 15 über eine zweite Länge L2 erstrecken. Die zwei Saugstückabschnitte 14 und 15 sind in radialen Richtungen zueinander verstellbar, was dadurch erreicht ist, dass in dem Übergangsbereich zwischen dem Basisteil 16 und den zwei Saugstückabschnitten 14 und 15 zwei einander gegenüberliegende kreisförmige Materialfreistellungen vorgesehen sind, welche zwei kreisförmigen Materialfreistellungen 17 je in eine schlitzförmige Materialfreistellung 18 übergehen, welche zwei schlitzförmigen Materialfreistellungen 18 bis zu der Saugöffnung 13 reichen. Im Bereich der zwei schlitzförmigen Materialfreistellungen 18 sind die zwei Saugstückabschnitte 14 und 15 zueinander benachbart.

In den zwei schlitzförmigen Materialfreistellungen 18 und in den zwei kreisförmigen Materialfreistellungen 17 ist je ein Dichtungsteil 19 und 20 vorgesehen. Der erste Dichtungsteil 19 ist aus der Figur 2 und der zweite Dichtungsteil 20 ist aus der Figur 1 ersichtlich. Die zwei Dichtungsteile 19 und 20 erstrecken sich je bis zu der Saugöffnung 13 und begrenzen daher ebenso die Saugöffnung 13 wie die zwei Saugstückabschnitte 14 und 15. Die Dichtungsteile 19 und 20 liegen einander diametral gegenüber und sind mit den zwei zueinander benachbarten Saugstückabschnitten 14 und 15 luftdicht verbunden. Die zwei Dichtungsteile 19 und 20 sind elastisch nachgiebig ausgebildet, bestehen also aus einem elastisch nachgiebigen Material. In dem hier vorliegenden Fall ist das erste Saugstück 8 mit seinen zwei Saugstückabschnitten 14 und 15 und seinen Dichtungsteilen 19 und 20 mit Hilfe von einem Zwei-Komponenten-Spritzgießverfahren hergestellt worden. Hierbei ist für den Basisteil 16 und die zwei Saugstückabschnitte 14 und 15 ein in seinem Endzustand relativ harter Kunststoff und für die zwei Dichtungsteile 19 und 20 ein in seinem Endzustand relativ weicher Kunststoff verwendet worden.

Wie aus der Figur 2 ersichtlich ist, weisen die zwei Saugstückabschnitte 14 und 15 des ersten Saugstücks 8 und die zwei Dichtungsteile 19 und 20 des ersten Saugstücks 8 je eine scharfe Begrenzungskante K zum Begrenzen der Saugöffnung 13 auf. Sämtliche Begrenzungskanten K weisen hierbei einen kreisbogenförmigen Verlauf auf. Die zwei Begrenzungskanten K der Saugstückabschnitte 14 und 15 und auch die Begrenzungskanten K der Dichtungsteile 19 und 20 weisen in diesem Fall einen diametrischen Abstand D1 von 3,4 mm auf. Erwähnt sei noch, dass der Durchmesser des Basisteils 16 des ersten Saugstücks 8 einen Durchmesser D2 von 10,0 mm aufweist. Weiters sei erwähnt, dass das bis zu der Saugöffnung 13 reichende Ende des ersten Saugstücks 8 an seiner Außenseite kegelstumpfförmig ausgebildet ist, wobei eine Öffnungswinkel β gewählt ist, der einen Wert von 33,40 ° aufweist.

Mit dem Körperpflegegerät 1 gemäß der Figur 1 und mit dem Saugstück 8 gemäß der Figur 2 ist sowohl eine gute Saugwirkung als auch zusätzlich eine gute Druckwirkung auf einen beim saugenden Zusammenwirken mit der Haut gebildeten Hauthöcker sichergestellt, so dass besonders gute Reinigungsergebnisse erzielt werden können.

Wie bereits erwähnt, kann bei einer Variante des Körperpflegegeräts 1 auch eine Variante eines ersten Saugstücks 8 vorgesehen sein, bei welcher Variante des ersten Saugstücks 8 zwei nebeneinanderliegende Saugöffnungen vorgesehen sind, die je für sich eine Ausbildung wie bei dem vorstehend beschriebenen ersten Saugstück 8 aufweisen.

Bei einer weiteren Ausführungsvariante kann ein wie vorstehend beschriebenes erstes Saugstück 8 auch so ausgebildet sein, dass im Inneren des Saugstücks eine Trennwand vorgesehen ist, die mit den beiden Dichtungsteilen verbunden ist und die im wesentlichen bis zu der Saugöffnung reicht, wobei dann die Saugöffnung in zwei Teilöffnungen unterteilt ist, in die je ein Hauthöcker eingesaugt werden kann, wobei dann mit Hilfe der Trennwand und jeweils einem verstellbaren Saugstückabschnitt eine Kraftwirkung auf die zwei gebildeten Hauthöcker ausgeübt werden kann.

## Patentansprüche

1. Körperpflegegerät mit einer Luftpumpe und mit einem Saugstück zum Ausüben einer Saugwirkung auf die Haut eines Menschen,
wobei das Saugstück mit der Luftpumpe luftleitend verbunden ist und
wobei das Saugstück mindestens eine umfangsseitig begrenzte Saugöffnung zum saugenden Zusammenwirken mit der Haut eines Menschen aufweist und
wobei das Saugstück im Bereich der Saugöffnung zum Bilden eines Hauthöckers bei einem saugenden Zusammenwirken mit der Haut ausgebildet ist und
wobei das Saugstück mindestens zwei bis zu der Saugöffnung sich erstreckende und die Saugöffnung begrenzende Saugstückabschnitte aufweist, welche zwei Saugstückabschnitte in radialen Richtungen verstellbar und zum Ausüben einer Kraftwirkung in radialen Richtungen auf einen bei einem saugenden Zusammenwirken mit der Haut gebildeten Hauthöcker ausgebildet sind, und
wobei das Saugstück mindestens zwei bis zu der Saugöffnung sich erstreckende und die Saugöffnung begrenzende und elastisch nachgiebig ausgebildete Dichtungsteile aufweist, wobei jeder Dichtungsteil zwischen zwei zueinander benachbarten Saugstückabschnitten liegt und mit den zwei zueinander benachbarten Saugstückabschnitten luftdicht verbunden ist.

2. Körperpflegegerät nach Anspruch 1,
wobei das Saugstück zwei einander diametral gegenüberliegende Saugstückabschnitte aufweist, die aus einem in Relation zu den elastisch nachgiebig ausgebildeten Dichtungsteilen harten Material bestehen.

3. Körperpflegegerät nach Anspruch 2,
wobei das Saugstück mit seinen zwei Saugstückabschnitten und seinen zwei Dichtungsteilen mit Hilfe von einem Zwei-Komponenten- Spritzgießverfahren hergestellt worden ist.

4. Körperpflegegerät nach Anspruch 2,
wobei die mindestens zwei Saugstückabschnitte des Saugstücks je eine scharfe Begrenzungskante zum Begrenzen der Saugöffnung aufweisen.

5. Körperpflegegerät nach Anspruch 4,
wobei die mindestens zwei Begrenzungskanten einen kreisbogenförmigen Verlauf aufweisen.

6. Körperpflegegerät nach Anspruch 5,
wobei die mindestens zwei Begrenzungskanten einen diametrischen Abstand in einem Bereich zwischen 3,0 mm und 4,0 mm aufweisen.

7. Körperpflegegerät nach Anspruch 6,
wobei die mindestens zwei Begrenzungskanten einen diametrischen Abstand von 3,4 mm aufweisen.

8. Saugstück für ein Körperpflegegerät,
wobei das Saugstück mindestens eine umfangsseitig begrenzte Saugöffnung zum saugenden Zusammenwirken mit der Haut eines Menschen aufweist und
wobei das Saugstück im Bereich der Saugöffnung zum Bilden eines Hauthöckers bei einem saugenden Zusammenwirken mit der Haut ausgebildet ist und
wobei das Saugstück mindestens zwei bis zu der Saugöffnung sich erstreckende und die Saugöffnung begrenzende Saugstückabschnitte aufweist, welche zwei Saugstückabschnitte in radialen Richtungen verstellbar und zum Ausüben einer Kraftwirkung in radialen Richtungen auf einen bei einem saugenden Zusammenwirken mit der Haut gebildeten Hauthöcker ausgebildet sind, und
wobei das Saugstück mindestens zwei bis zu der Saugöffnung sich erstreckende und die Saugöffnung begrenzende und elastisch nachgiebig ausgebildete Dichtungsteile aufweist, wobei jeder Dichtungsteil zwischen zwei zueinander benachbarten Saugstückabschnitten liegt und mit den zwei zueinander benachbarten Saugstückabschnitten luftdicht verbunden ist.

9. Saugstück nach Anspruch 8,
wobei das Saugstück zwei einander diametral gegenüberliegende Saugstückabschnitte aufweist, die aus einem in Relation zu den elastisch nachgiebig ausgebildeten Dichtungsteilen harten Material bestehen.

10. Saugstück nach Anspruch 9,
wobei das Saugstück mit seinen zwei Saugstückabschnitten und seinen zwei Dichtungsteilen mit Hilfe von einem Zwei-Komponenten- Spritzgießverfahren hergestellt worden ist.

11. Saugstück nach Anspruch 8,
wobei die mindestens zwei Saugstückabschnitte des Saugstücks je eine scharfe Begrenzungskante zum Begrenzen der Saugöffnung aufweisen.

12. Saugstück nach Anspruch 11,
wobei die mindestens zwei Begrenzungskanten einen kreisbogenförmigen Verlauf aufweisen.

13. Saugstück nach Anspruch 12,
wobei die mindestens zwei Begrenzungskanten einen diametrischen Abstand in einem Bereich zwischen 3,0 mm und 4,0 mm aufweisen.

14. Saugstück nach Anspruch 13,
wobei die mindestens zwei Begrenzungskanten einen diametrischen Abstand von 3,4 mm aufweisen.
